# EUROPÄISCHE PATENTSCHRIFT

(11) **EP 2 565 259 B1**
(45) Veröffentlichungstag und Bekanntmachung des Hinweises auf die Patenterteilung: **18.02.2015**
(21) Anmeldenummer: 11179779.1
(22) Anmeldetag: 01.09.2011
(51) Int. Cl.: C12M 1/107

(54) **Verfahren und Vorrichtung zur Biogaserzeugung**
Method and device for biogas production
Procédé et dispositif destinés à la production de biogaz

(43) Veröffentlichungstag der Anmeldung: 06.03.2013
(73) Patentinhaber: Conviotec GmbH, 24941 Flensburg (DE)
(72) Erfinder: Schneider, Dr., Holger, 24941 Flensburg (DE)
(74) Vertreter: Hansen, Jochen

(56) Entgegenhaltungen:
- WO-A1-2008/099227
- CH-A5- 688 737
- US-B1- 6 660 518

## Beschreibung

Die Erfindung betrifft ein Verfahren zur Erzeugung von Biogas, speziell von Methan, aus biologischen Materialien und genauer ein Verfahren zur Fermentation eines fließfähigen Substrats unter Verwendung eines Reaktors, mindestens aufweisend:
□ einen Zulauf (1),
□ einem Ablauf (3),
□ eine Mehrzahl von Trennwänden (6), die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,

Die Erfindung betrifft im gleichen Maße auch einen Reaktor zur Fermentation eines fließfähigen Substrats, wie er für das erfindungsgemäße Verfahren zur Anwendung kommt.

Der Entstehungsprozess von Biogas aus biologischem Ausgangsmaterial kann im Wesentlichen in vier Schritte unterteilt werden:
▪ Im ersten Schritt, der "Hydrolyse", werden die komplexen Verbindungen des Substratmaterials (z.B. Kohlenhydrate, Eiweiße, Fette) in einfachere organische Verbindungen (z.B. Aminosäuren, Zucker, Fettsäuren) zerlegt. Die daran beteiligten Bakterien setzen hierzu Enzyme frei, die das Material auf biologischem Wege zersetzen. Grundsätzlich kann dieser erste Schritt, die Hydrolyse, anaerob wie auch aerob erfolgen.
▪ Im zweiten Schritt werden dann die gebildeten Zwischenprodukte innerhalb der so genannten "Versäuerungsphase" (Acidogenese) durch säurebildende Bakterien weiter zu niederen Fettsäuren (z.B. Essig-, Propion- und Buttersäure) sowie Kohlendioxid (CO2) und Wasserstoff abgebaut. Daneben werden auch geringe Mengen an Milchsäure und Alkoholen gebildet.
▪ Im dritten Schritt werden anschließend die Vorprodukte innerhalb der "Essigsäurebildung" (Acetogenese), durch Bakterien zu Vorläufersubstanzen des Biogases (Essigsäure, Wasserstoff und Kohlendioxid) umgesetzt.
▪ In dem letzten Schritt der Biogasherstellung, der "Methanogenese", wird ebenfalls durch Bakterien aus den Produkten der Acetogenese das Methan gebildet.

Laufen die vier Abbauschritte gemeinsam in einem Fermenter ab, spricht man von einstufigen Anlagen. Da die Bakterien der einzelnen Stufen aber unterschiedliche Anforderungen an den Lebensraum stellen, kann eine räumliche Trennung der Abbaustufen von Vorteil sein.

Aus der WO 2006 / 124781 ist eine Biogasanlage für die anaerobe Fermentation bekannt, bei der ein zur Anwendung kommender Reaktor in verschiedene, fest konfektionierte Kammern aufgeteilt ist und in denen die im Absatz [0003] beschriebenen Reaktionen stattfinden.

In der US 2007 / 025 69 71 A1 wird eine transportable Biogasanlage zur anaeroben Fermentation beansprucht, die aus mehreren in ihrer Größe jeweils fest konfektionierten, flexiblen, blasenartigen Kammern besteht.

Aus CH-A5-688737 ist eine Biogasanlage mit einer Mehrzahl von Trennwänden bekannt, die mindestens das für das Substrat bestimmte Innenvolumen des Reaktors in eine Mehrzahl von Kompartimenten unterteilen.

Beiden bekannten Biogasanlagen ist als Nachteil gemeinsam, dass sie in ihrer starren Konfektionierung in einem besonderen Maße auf die Störanfälligkeit der Methanbakterien ausgerichtet sein müssen. Es ist ihnen ferner verfahrenseigen, dass sie die Gase, die während der Prozessschritte erzeugt werden, im Gasraum über dem Fermenter vermischen. Als Grund für die Störanfälligkeit der aus dem Stand der Technik bekannten Gasanlagen wurde ferner erkannt, dass die Methanbakterien aus der Liste aller an der anaeroben Fermentation planmäßig beteiligten Bakterien am empfindlichsten auf Störungen reagieren und sich überdies nur sehr langsam vermehren. Infolgedessen werden die Milieubedingungen innerhalb der gesamten aus dem Stand der Technik bekannten Reaktoren an die Methanbakterien angepasst.

Der oben genannten räumlichen Trennung der Abbaustufen sind jedoch Grenzen insofern gesetzt, da beispielsweise die Bakterien der Acetogenese auf eine Lebensgemeinschaft mit denen der Methanogenese angewiesen sein können. Der Grund für diese Abhängigkeit liegt darin, dass die Bakterien der Acetogenese einen zu hohen Wasserstoffgehalt nicht vertragen.

Ein besonderes Problem von solchen dem Stand der Technik zuzurechnenden transportablen Anlagen mit kleinen Fermentervolumina ist ihre besonders hohe Störanfälligkeit; Veränderungen der Substratzusammensetzung, welche bei biologischen Materialien naturgemäß gegeben sind und Unregelmäßigkeiten in der Substratzuführung führen hier sehr schnell zu Änderungen des pH-Wertes und zur Verschiebungen der mikrobiellen Besiedelung und damit zur Instabilität des Systems.

Diesen Problemen tritt man zurzeit durch die Verwendung nicht transportabler großtechnischer Biogasanlagen, welche über kontinuierlich durchmischte Reaktoren (CSTR - continuous stirred tank reactor) verfügen, mit bis zu mehreren tausend Kubikmetern Arbeitsvolumen und langen Verweilzeiten entgegen. Auch diese Systeme zeigen noch eine erhebliche Anfälligkeit gegen Änderungen der Substratzusammensetzung.

In einem kontinuierlich durchmischten Reaktor (CSTR) sind idealerweise an allen Stellen im Reaktor gleiche Fermentations- und Strömungsbedingungen. Dadurch wirken sich Störungen bedauerlicherweise sofort auf den gesamten Reaktorinhalt aus. Dieses Prozessrisiko wird durch sehr niedrige Raumbelastungen (geringe Substratzufuhr pro Zeiteinheit) des Reaktors möglichst gering gehalten.

Der Erfindung liegt daher die Aufgabe zugrunde, der Öffentlichkeit einen Reaktor zur Erzeugung von Biogas zur Verfügung zu stellen, in dem der Biogasprozess bei Veränderung der Substratzusammensetzung und der Substratzuführung stabil bleibt, hohe Raum-Zeit-Ausbeuten ermöglicht, mit kurzen Verweilzeiten effektiv arbeitet, auch in kleinen Fermentervolumina beherrschbar ist und selbst in transportablen Behältern noch mit geringem Wartungs- und Überwachungsaufwand verfahrensstabil abläuft. Ein ganz besonderes Augenmerk der Aufgabe richtet sich überdies auf die Gewährleistung eines möglichst hohen Brennwertes des in dem Reaktor erzeugten Brennwertes. Der Erfindung liegt im gleichen Maße auch die Aufgabe zugrunde, ein entsprechendes Verfahren zur Erzeugung von Biogas unter Nutzung des aufgabengemäßen Reaktors vorzuschlagen.

Die Aufgabe wird erfindungsgemäß durch ein Verfahren zur Fermentation eines fließfähigen Substrats mit definiertem Trockensubstanzgehalt unter Verwendung eines Reaktors gelöst, wobei das Verfahren mindestens die Verfahrensschritte aufweist:
□ Zuführen des Substrates durch mindestens einen Zulauf (1) des Reaktors,
□ Hindurchführen des Substrates durch den Reaktor, wobei der Reaktor eine Mehrzahl von Trennwänden (6) aufweist, die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,
□ Abführen des fermentierten Substrates aus mindestens einen Ablauf (3) des Reaktors,
wobei das hier vorgeschlagene Verfahren gekennzeichnet wird dadurch, dass
▪ zur Erhöhung oder Reduktion des Verhältnisses
   - des Volumens der in die eine Richtung vom Substrat durchströmten Kammern (8 (i) - 8 (iv))
   - zu dem Volumen der in die andere Richtung vom Substrat durchströmten Kammern (9 (i) - 9 (iv))
   zumindest ein Teil der Trennwände (6) in ihrer räumlichen Lage und/oder Position und/oder Ausdehnung bewegt werden,
   wobei die Bewegung und/oder Ausdehnung der Trennwände (6) in Abhängigkeit vom Trockensubstanzgehalt des fließfähigen Substrats gesteuert wird,
▪ jedes Kompartiment (7 (i) - 7 (iv)) ein für das Substrat bestimmtes Innenvolumen und einen zugeordneten Gasraum (5 (i) - 5 (iv)) aufweist,
▪ mindestens ein Gasraum von mindestens einem Kompartiment, ausgesucht aus allen Gasräumen (5 (i) - 5 (iv)) aller Kompartimente (7 (i) - 7 (iv)), getrennt ausgebildet ist von dem restlichen Gasraum oder den restlichen Gasräumen (5 (i) - 5 (iv)) der restlichen Kompartimente (7 (i) - 7 (iv)),
und wobei das hier vorgeschlagene Verfahren in kennzeichnender Weise mindestens die folgenden weiteren Verfahrensschritte aufweist:
▪ Abführen von Prozessgas aus dem mindestens einen Gasraum, getrennt ausgebildet von dem restlichen Gasraum oder den restlichen Gasräumen (5 (i) - 5 (iv)) der restlichen Kompartimente (7 (i) - 7 (iv)),
▪ Zuführen von zuvor abgeführtem Prozessgas und/oder Fremdgas in das für das Substrat bestimmte Innenvolumen von mindestens einem der ausgebildeten Kompartimente (7 (i) - 7 (iv)).

Erfindungsgemäß weist das Verfahren mindestens die weiteren Verfahrensschritte
▪ Abführen von Prozessgas aus dem mindestens einen Gasraum, getrennt ausgebildet von dem restlichen Gasraum oder den restlichen Gasräumen (5 (i) - 5 (iv)) der restlichen Kompartimente (7 (i) - 7 (iv))
   und
▪ Zuführen von zuvor abgeführtem Prozessgas und/oder Fremdgas in das für das Substrat bestimmte Innenvolumen von mindestens einem der ausgebildeten Kompartimente (7 (i) - 7 (iv))
   Auf. Als getrennt ausgebildet gilt der mindestens eine Gasraum von dem restlichen Gasraum oder den restlichen Gasräumen (5 (i) - 5 (iv)) der restlichen Kompartimente (7 (i) - 7 (iv)) dann, wenn ein Gasaustausch zwischen diesem mindestens einen Gasraum und dem restlichen Gasraum bzw. den restlichen Gasräumen (5 (i) - 5 (iv)) der restlichen Kompartimente (7 (i) - 7 (iv)) nicht möglich ist. Eine solche Trennung kann durch eine gasdichte Wand bzw. ein gasdichtes Schott erfolgen, sie kann genauso durch eine vollständige räumliche Trennung erfolgen - weitere Umsetzungen für eine solche Trennung sind vorstellbar, so dass die genannten Beispiele keinen abschließenden Charakter aufweisen sollen. Im Rahmen der beiden vorbenannten zwei weiteren Verfahrensschritte und genauso und insbesondere im Rahmen des dafür benutzten Reaktors gilt es als bevorzugt,
▪ wenn jedes Kompartiment (7 (i) - 7 (iv)) ein für das Substrat bestimmtes Innenvolumen und einen eigenen Gasraum (5 (i) - 5 (iv)) aufweist, wobei dieser eigene Gasraum von den Gasräumen der anderen Kompartimente (7 (i) - 7 (iv)) getrennt ausgebildet ist,
▪ und wenn dabei jeder Gasraum (5 (i) - 5 (iv)) von jedem Kompartiment (7 (i) - 7 (iv)) einen ihm, bevorzugt ausschließlich ihm zugeordneten Gasausgang (2 (i) - 2 (iv)) zur Abführen von Prozessgas aus diesem Gasraum (5 (i) - 5 (iv)) aufweist.

Ferner ist es bevorzugt, wenn jedes Kompartiment (7 (i) - 7 (iv)) ein ihm, bevorzugt ausschließlich ihm zugeordneten Gaseintritt (10 (i) - 10 (iv)) zur Zuführung von zuvor abgeführtem Prozessgas und/oder von Fremdgas in das für das Substrat bestimmte Innenvolumen von diesem Kompartiment (7 (i) - 7 (iv)) aufweist. Die Zuführung von zuvor abgeführtem Prozessgas und/oder von Fremdgas erfolgt bevorzugt so, dass dieses Prozessgas und/oder Fremdgas, gebündelt - beispielsweise durch eine Düse, in einem Gasstrom in das für das Substrat bestimmte Innenvolumen der Kompartimente (7 (i) - 7 (iv)) eingeleitet wird, wobei dieser Gasstrom bevorzugt in Gegenstromrichtung des Substrates ausgerichtet ist. Das bedeutet, wird das Prozessgas und/oder Fremdgas in den vom Substrat abwärts durchströmten Kammern (8 (i) - 8 (iv)) zugeführt, ist der Gasstrom bevorzugt aufwärts strömend ausgerichtet, wird das Prozessgas und/oder Fremdgas in den vom Substrat aufwärts durchströmten Kammern (9 (i) - 9 (iv)) zugeführt, ist der Gasstrom bevorzugt abwärts strömend ausgerichtet. Ganz besonders bevorzugt erfolgt die Zuführung von Prozessgas und/oder Fremdgas am Boden der vom Substrat abwärts durchströmten Kammern (8 (i) - 8 (iv)), wozu der entsprechend ausgebildete Reaktor dadurch gekennzeichnet ist, dass der-jeweilige - Gaseintritt (10 (i) - 10 (iv)) zur Zuführung von Prozessgas und/oder Fremdgas am Boden der vom Substrat abwärts durchströmten Kammern (8 (i) - 8 (iv)) ausgebildet ist. Ganz besonders bevorzugt wird Prozessgas jeweils des nachfolgenden Kompartiments (7 (i) - 7 (iv)) in das Innenvolumen des vorherigen Kompartiments (7 (i) - 7 (iv)) eingeführt, gegebenenfalls ergänzt durch Frisch- und/oder Fremdgas. Die bevorzugte Rückführung von Prozessgas in das Innenvolumen vorgeschalteter Kompartimente (7 (i) - 7 (iv)) führt gemäß den Erkenntnissen der Erfinderschaft zu Biogas mit einem besonders hohen Wobbeindex (korrigierter Heizwert), der sich als Quotient des Heizwertes des entsprechenden Brenn- bzw. Biogases und der Wurzel aus dem Dichteverhältnis von diesem Brenn- bzw. Biogas und Luft berechnet. In einem ganz besonders bevorzugten Aspekt der Erfindung wird ein Sauerstoff enthaltendes Gas, wie beispielsweise reiner Sauerstoff oder wie Luft, als Fremdgas in das Innenvolumen des ersten vom Substrat passierten Kompartiments (7 (i)) eingeführt.

Damit kommt der anaerobe Biogasprozess innerhalb dieses Kompartiments (7 (i)) zum Erliegen. Es wird kein Methan gebildet. Dafür werden aerobe Bakterien aktiv, die mit hoher Geschwindigkeit organische Materialien mit polymeren Strukturen (wie beispielsweise Polysaccharide, Eiweiße, Fette) in Monomere zerlegen und den nachgeschalteten Biogasprozess - in den Kammern der nachfolgenden Kompartiments (7 (ii) - 7 (iv)) - extrem beschleunigen. Eine Verdopplung bis Verdreifachung der Biogasbildungsrate wurde in dieser Erfindung zugrundeliegenden Laboruntersuchungen je nach Substrat nachgewiesen. Stoffe, die entsprechend des bisherigen Standes der Technik als nicht oder schwer abbaubar galten, wie zum Beispiel das so genannte "Black Water" aus der Palmölindustrie, können so für den Biogasprozess zugänglich gemacht werden. Die Luft- oder Sauerstoffzufuhr wird sinnvollerweise so dosiert, dass im Gasraum (5 (i)) des ersten Kompartiments (7 (i)) kein Sauerstoff nachweisbar ist. Der Sauerstoff wird von den Bakterien komplett für die Hydrolyse der Polymere genutzt. Bei der Hydrolyse entsteht ein Gasgemisch aus Kohlendioxid und Wasserstoff, das entweder im weiteren Biogasprozess verworfen oder in den nachfolgenden Kompartimenten (7 (ii) - 7 (iv)) zugeführt und für die Methanproduktion genutzt wird.

Bevorzugt ist der für das vorgeschlagene Verfahren zur Anwendung kommende Reaktor ein aus Stahl gefertigter Behälter in kubischer oder zylindrischer Form, bei letztgenannter Bauform mit runder oder elliptischer Form. Zumindest für kleinere Bauformen, wie beispielsweise Versuchsreaktoren, eignet sich als Baumaterial insbesondere und bevorzugt Acrylglas, Kunststoff und Faserverstärkter Kunststoff, für Großreaktoren bieten sich bevorzugt Beton, Stahl und Stahlbeton hinsichtlich des Boden und für die Seitenwände sowie Stahl und Faserverstärkte Kunststoffe für das Dach an, ohne auf die Bauformen und/oder die genannten Baumaterialien im Sinne der vorliegenden Erfindung beschränkt zu sein. Hinsichtlich des Innenvolumens des Reaktors sind Größenordnungen von 4 Liter für Versuchsreaktoren bis 200 m³ für Großreaktoren bevorzugt und vorstellbar.

In einer bevorzugten Ausführungsform des vorgeschlagenen Verfahrens weist der dabei verwendete Reaktor in einer gleichsam bevorzugten Ausführung entlang seiner Längsachse eine Mehrzahl nebeneinander positionierter Kompartimente (7 (i) - 7 (iv)) auf. In zahlreichen der Erfindung vorausgegangenen Versuchen wurden bei dem erfindungsgemäßen Verfahren insbesondere solche Reaktoren verwendet, bei denen jedes einzelne Kompartiment (7 (i) - 7 (iv)) jeweils in zwei gegenläufig vom Substrat durchströmte Kammern (8 (i) - 8 (iv); 9 (i) - 9 (iv)) unterteilt ist, von denen die Kammern (8 (i) - 8 (iv)) - pro Kompartiment bevorzugt die jeweils zuerst vom Substrat durchströmte Kammer - vom Substrat abwärts und die Kammern (9 (i) - 9 (iv)) - pro Kompartiment die dann jeweils zuletzt vom Substrat durchströmte Kammer - vom Substrat aufwärts durchströmt werden. Derartige Reaktoren gelten gleichsam als bevorzugt im Sinne der Erfindung. Dabei können ganz besonders bevorzugt in Abhängigkeit vom Trockensubstanzgehalt des fließfähigen Substrats zumindest ein Teil der Trennwände (6) entlang der Richtung der Längsachse des Reaktors verschoben werden. Die Unterteilung des Reaktors in eine Mehrzahl von Kompartimenten (7 (i) - 7 (iv)) und die Unterteilung jedes einzelnen Kompartiments (7 (i) - 7 (iv)) jeweils in mindestens zwei Kammern (8 (i) - 8 (iv); 9 (i) - 9 (iv)) geschieht dabei mit einer Mehrzahl von Trennwänden (6), die bevorzugt vertikal ausgerichtet sind.

Die hier vorgeschlagenen Verfahren setzen jedoch keineswegs eine Bauform der zu verwendenden Reaktoren mit entlang der Längsachse des Reaktors nebeneinander positionierten Kompartimenten (7 (i) - 7 (iv)) und mit entlang der Richtung der Längsachse des Reaktors verschiebbaren vertikal ausgerichtet Trennwänden (6) voraus. Genauso vorstellbar sind beispielsweise übereinander liegende Kompartimente (7 (i) - 7 (iv)) mit bevorzugt horizontal ausgerichteten Trennwänden (6).

Hinsichtlich der Trennwände (6) ist neben deren Verschiebung als Bewegungsform auch deren Drehung, Kippen bzw. Klappen, Aus- und Einfahren sowie ein sich räumliches Ausdehnen und sich Verjüngen jeweils in Abhängigkeit vom Trockensubstanzgehalt des Substrats vorstellbar, wobei insbesondere das Drehen, Kippen bzw. Klappen der Trennwände als neben dem Verschieben besonders bevorzugte Ausführungsformen hinsichtlich der Bewegung der Trennwände (6) sind.

Überraschenderweise hat sich gezeigt, dass sich ein Zustand der Autoimmobilisierung der Bakterien in den jeweiligen aufwärts durchströmten Kammern (9 (i) - 9 (iv)) der einzelnen Kompartimente (7 (i) - 7 (iv)) erreichen lässt, wenn der Abstand der Trennwände (6) in erfindungsgemäßer Art und Weise an den Gehalt der Trockensubstanz des verwendeten Substrates angepasst wird. Dabei wird der Trockensubstanzgehalt des Substrats bevorzugt im Zulauf (1) zum Reaktor bemessen. Genauso ist eine Bestimmung des Trockensubstanzgehalts des Substrates im Ablauf (3) möglich, setzt aber dann genauere Kenntnisse zum Prozessverhalten des Reaktors voraus. Es hat sich ferner gezeigt, dass in dem Reaktor eine durch den biologischen Prozessverlauf bedingte Gastrennung in Methan, Wasserstoff und CO₂ in gewissem Umfang selbstständig erfolgt.

Bei der erfindungsgemäßen Anpassung des Abstands der Trennwände (6) an den Gehalt der Trockensubstanz des verwendeten Substrates kommt es so zu einer Entkopplung der hydraulischen von der Feststoffverweilzeit. Durch die gezielte Modifikation der Kammern (8 (i) - 8 (iv); 9 (i) - 9 (iv)) der Kompartimente (7 (i) - 7 (iv)) ergibt sich eine vollkommen neue Methode zur Steuerung des Prozesses der Biogas-Produktion.

Tabelle 1 zeigt für besonders stabil ablaufende Biogas-Herstellungsabläufe das jeweilige und im Sinne der vorliegenden Erfindung besonders bevorzugte Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) in Abhängigkeit vom Trockensubstanzgehaltes des Substrates.

**Tabelle 1**

| Trockensubstanzgehalt in Gew.-% | Verhältnis des jeweiligen Volumens der abwärts vom Substrat durchströmten Kammern (8 (i) - 8 (iv)) zu dem jeweiligen Volumen der aufwärts vom Substrat durchströmten Kammern (9 (i) - 9 (iv)) | Siehe dazu Figur |
|---|---|---|
| <2% | 1:3 | 2a |
| 2-5% | 1:2 | 2b |
| 5-10% | 1:1 | 2c |
| 10-15% | 2:1 | 2d |
| 15-20% | 3:1 | 2e |

Der Tabelle lässt sich entnehmen, dass bei dem vorgeschlagenen Verfahren bevorzugt das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) derart eingestellt wird, dass es mit steigendem Trockensubstanzgehalt des Substrates zunimmt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von < 2 Gew.-% in einem Bereich von [1 : 3,5] bis [1 : größer 2,5] liegt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von 2 Gew.-% bis 5 Gew.-% in einem Bereich von [1 : 2,5] bis [1 : größer 1,5] liegt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von 5 Gew.-% bis 10 Gew.-% in einem Bereich von [1 : 1,5] bis [kleiner 1,5 : 1] liegt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von 10 Gew.-% bis 15 Gew.-% in einem Bereich von [größer 1,5 : 1] bis [2,5 : 1] liegt.

Insbesondere ist es bevorzugt, wenn bei dem vorgeschlagenen Verfahren das Verhältnis des jeweiligen Volumens der abwärts vom Substrat durchströmten Kammern (8 (i) - 8 (iv)) zu dem Volumen der aufwärts vom Substrat durchströmten Kammern (9 (i) - 9 (iv)) bei einem Trockensubstanzgehalt von 15 Gew.-% bis 20 Gew.-% in einem Bereich von [größer 2,5 : 1] bis [3,5 : 1] liegt.

Die Erfindung betrifft im gleichen Maße auch einen Reaktor, wie er für das erfindungsgemäße Verfahren in mindestens einer der bevorzugten Ausführungsformen zur Anwendung kommt. Dabei weist ein solcher Reaktor zur Fermentation eines fließfähigen Substrats mit definiertem Trockensubstanzgehalt, mindestens auf:
□ einen Zulauf (1),
□ einen Ablauf (3),
□ eine Mehrzahl von Trennwänden (6), die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,
wobei der hier vorgeschlagene Reaktor gekennzeichnet wird dadurch, dass
▪ zur Erhöhung oder Reduktion des Verhältnisses
   - des Volumens der in die eine Richtung vom Substrat durchströmten Kammern (8 (i) - 8 (iv)
   - zu dem Volumen der in die andere Richtung vom Substrat durchströmten Kammern (9 (i) - 9 (iv)
   zumindest ein Teil der Trennwände (6) in ihrer räumlichen Lage und/oder Position beweglich angeordnet sind,
   und dass der Reaktor mindestens eine Steuerung zur Bewegung der Trennwände (6) in Abhängigkeit vom Trockensubstanzgehalt des fließfähigen Substrats aufweist,
▪ jedes Kompartiment (7 (i) - 7 (iv)) ein für das Substrat bestimmte Innenvolumen und einen Gasraum (5 (i) - 5 (iv)) aufweist,
▪ mindestens ein Gasraum von mindestens einem Kompartiment, ausgesucht aus allen Gasräumen (5 (i) - 5 (iv)) aller Kompartimente (7 (i) - 7 (iv)), getrennt ausgebildet ist von dem restlichen Gasraum oder den restlichen Gasräumen (5 (i) - 5 (iv)) der restlichen Kompartimente (7 (i) - 7 (iv)),
▪ der mindestens eine Gasraum einen Gasausgang zur Abführung von Prozessgas aufweist,
▪ mindestens ein Kompartiment (7 (i) - 7 (iv)) einen Gaseintritt (10 (i) - 10 (iv)) zur Zuführung von Prozessgas und/oder Fremdgas aufweist.

Ganz besonders bevorzugt gilt dabei ein Reaktor,
▪ wenn jedes Kompartiment (7 (i) - 7 (iv)) ein für das Substrat bestimmtes Innenvolumen und einen eigenen Gasraum (5 (i) - 5 (iv)) aufweist, wobei dieser eigene Gasraum von den Gasräumen der anderen Kompartimente (7 (i) - 7 (iv)) getrennt ausgebildet ist,
▪ und wenn dabei jeder Gasraum (5 (i) - 5 (iv)) von jedem Kompartiment (7 (i) - 7 (iv)) einen ihm, bevorzugt ausschließlich ihm zugeordneten Gasausgang (2 (i) - 2 (iv)) zur Abführen von Prozessgas aus diesem Gasraum (5 (i) - 5 (iv)) aufweist.

In einer bevorzugten Ausführungsform erstrecken sich ferner die Trennwände (6) über die gesamte Breite des Reaktors.

Die Anordnung der Trennwände (6) erfolgt sinnvollerweise derart, dass die Bildung von Rinnsalen und/oder Pfropfen innerhalb des Reaktors vermieden wird und ein optimaler Fluss durch den Reaktor dauerhaft gewährleistet werden kann.

Die Böden der einzelnen Kompartimente (7 (i) - 7 (iv)) können verschieden gestaltet sein, sie können beispielsweise rund sein oder gerade mit oder ohne Neigung. Sie können neben dem jeweiligen Gaseintritt (10 (i) - 10 (iv)) auch mit einer oder mehreren Entnahmestellen für das in den Reaktor eingegebene Substrat ausgestattet sein, so dass es möglich ist, Substrat an verschiedenen Stellen aus dem Reaktor zu entnehmen und an anderer Stelle wieder in den Reaktor einzubringen (recyclieren). Zur Entnahme des Substrats kann es vorteilhaft sein, eine Pumpe einzusetzen. Insbesondere eine Monopumpe, die über verschiedene Ventile mit allen intermediären Ablauf- und Zulaufstellen geschaltet ist, kann zu Zwecken des Recyclierens eingesetzt werden.

Der Durchfluss durch den Reaktor wird durch die Anordnung von Zulauf (1) und Ablauf (3) sowie nach dem Prinzip der kommunizierenden Röhren rein hydraulisch vollzogen oder über eine oder mehrere Pumpe unterstützt.

Die einzelnen Kompartimente (7 (i) - 7 (iv)) weisen jeweils einen Gasraum (5 (i) - 5 (iv)) auf, von denen erfindungsgemäß mindestens ein Gasraum von mindestens einem Kompartiment, ausgesucht aus allen Gasräumen (5 (i) - 5 (iv)) aller Kompartimente (7 (i) - 7 (iv)), getrennt ausgebildet ist von dem restlichen Gasraum oder den restlichen Gasräumen (5 (i) - 5 (iv)) der restlichen Kompartimente (7 (i) - 7 (iv)). Somit ist es möglich, dass die restlichen Gasräume der restlichen Kompartimente (7 (i) - 7 (iv)) miteinander verbunden sind. Bevorzugt sind jedoch sämtliche Gasräumen (5 (i) - 5 (iv)) aller Kompartimente (7 (i) - 7 (iv)) voneinander getrennt ausgebildet. Eine Entnahme von Prozessgas kann zum einen über den Gasausgang des mindestens einen getrennt ausgebildeten Gasraums und über einen Gasausgang der restlichen, miteinander verbundenen Gasräume erfolgen. Bevorzugt verfügt jeder der voneinander getrennt ausgebildeten Gasräume (5 (i) - 5 (iv)) über einen eigenen Gasausgang 2 (i) bis 2 (iv).

Der erfindungsgemäße Reaktor kann von seinen Abmaßen so gestaltet werden, dass er in einen Container eingelassen werden kann und somit transportabel ist.

Vor dem Eintrag in den erfindungsgemäßen Reaktor können die zu vergärenden Materialien auf eine geeignet Art und Weise derart vorbehandelt werden, dass die durchschnittliche Partikelgröße ≤ 5mm beträgt.

Der erfindungsgemäße Reaktor/das erfindungsgemäße Verfahren ermöglicht die anaerobe bzw. anaerobe/aerobe Vergärung bevorzugt von Materialien mit einem Trockenmassegehalt von 2-20% und einem CSB von 3000 mg/l bis 500.000 mg/l. Die Rückführungsquote liegt bevorzugt zwischen 10% und 50%. Für einen Biogas-Erzeugungsprozess in einem Reaktor von beispielsweise 50 m³ Innenvolumen aller Kompartimente (7 (i) - 7 (iv)) zusammen liegt der rückgeführte Volumenstrom je nach Substrat und Leistung zwischen 0,5 m³/h und 15 m³/h.

Der Anteil der nicht vergärbaren Substanzen in dem zugeführten Substrat überschreitet bevorzugt nicht einen Anteil von 20 Gew.-% in der Trockenmasse. Der Aufschluss der zugeführten Substrate kann durch das Einführen von Gasen in den vorgesehen bodennahen Gaseintritten 10 (i) bis 10 (iv) beschleunigt und vervollständigt werden.

Die in Beschreibung und Patentansprüchen gemachten Angaben zu Gew.-% (Gewichts-%) beziehen sich jeweils auf das "atro"-Gewicht, d.h. absolut trockene Gewichtsteile.

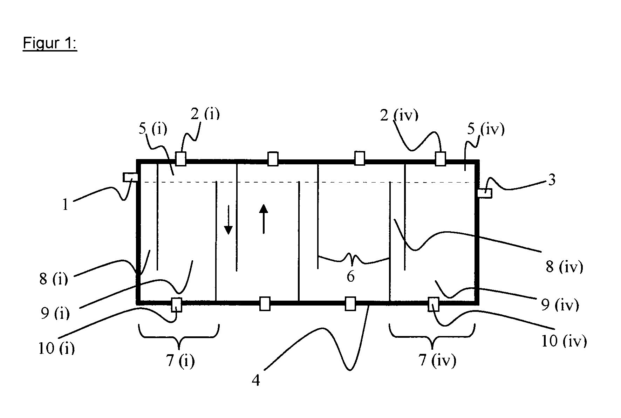

Figur 1 stellt eine mögliche Ausführungsform des hier vorgeschlagenen Reaktors zur Fermentation eines fließfähigen Substrats mit definiertem Trockensubstanzgehalt dar. Der Reaktor ist dabei ein kubischer Behälter mit horizontal ausgerichtetem rechteckigem Boden (4), vertikalen rechteckigen Kopf- und Seitenwänden und einem horizontal ausgerichtetem Dach. Der Reaktor weist im oberen Bereich der einen Kopfwand eine Zulauf (1) und im oberen Bereich der gegenüberliegenden Kopfwand einen Ablauf (3) auf, durch die dem Reaktor Substrat zugeführt und entnommen werden kann. Über drei - im vorliegenden Fall nicht bewegliche - vertikal vom Boden (4) in den Innenraum des Reaktors hineinragende Trennwände (6) ist der Innenraum des Reaktors in vier Kompartimente (7 (i) - 7 (iv)) unterteilt. Über vier bewegliche - hier entlang der Richtung der Längsachse des Reaktors verschiebbare - Trennwände (6) ist jedes einzelne Kompartiment (7 (i) - 7 (iv)) jeweils in genau zwei gegenläufig vom Substrat durchströmte Kammern (8 (i) - 8 (iv); 9 (i) - 9 (iv)) unterteilt. Dabei werden die vom Substart pro Kompartiment (7 (i) - 7 (iv)) zuerst durchströmten Kammern (8 (i) - 8 (iv)) vom Substrat abwärts gerichtet durchströmt, die vom Substart pro Kompartiment (7 (i) - 7 (iv)) zuletzt durchströmten Kammern (9 (i) - 9 (iv)) vom Substrat aufwärts gerichtet durchströmt. Am Boden der vom Substrat hier aufwärts durchströmten Kammern (9 (i) - 9 (iv)) ist jeweils ein Gaseintritt (10 (i) - 10 (iv)) zur Zuführung von Prozessgas und/oder von Fremdgas vorgesehen. Noch besser könnte eine Positionierung der Gaseintritt (10 (i) - 10 (iv)) jeweils nach links verrückt am Boden der vom Substrat hier abwärts durchströmten Kammern (8 (i) - 8 (iv)) sein. Im oberen Bereich eines jeden Kompartiments (7 (i) - 7 (iv)) ist ein jeweiliger Gasraum (5 (i) - 5 (iv)) ausgebildet mit einem jeweils eigenen Gasausgang (2 (i) - 2 (iv)), über den die bei der Fermentation entstehenden Prozessgase abgeführt werden können.

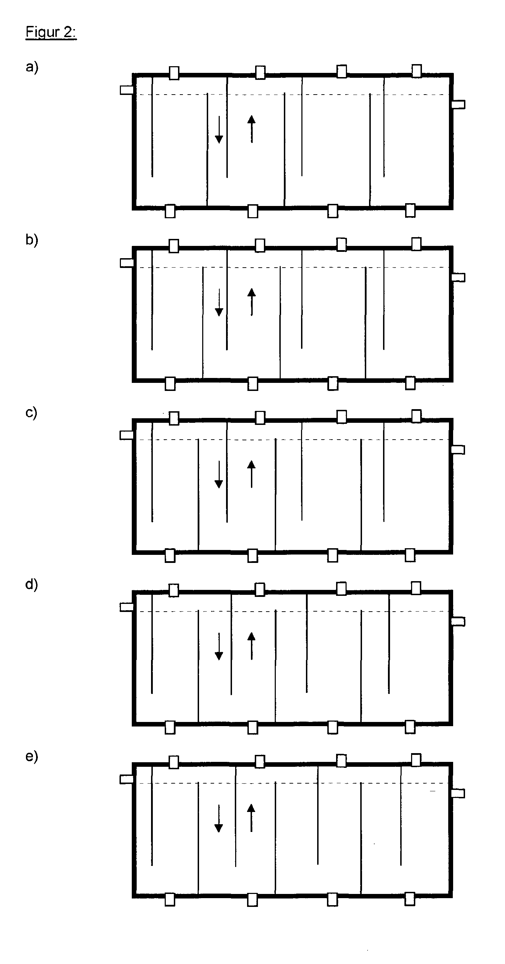

Figur 2 mit ihren Teilfiguren a. bis e. zeigt zur Verdeutlichung die durch Verschiebung der Trennwände (6) beeinflussbaren Verhältnisse des jeweiligen Volumens der abwärts vom Substrat durchströmten Kammern (8 (i) - 8 (iv)) zu dem jeweiligen Volumen der aufwärts vom Substrat durchströmten Kammern (9 (i) - 9 (iv)) für die in der Tabelle 1 dargestellten Fälle.

Zur Demonstration der erfindungsgemäßen Lehre wurden im Folgenden die Allgemeinheit der Lehre nicht einschränkenden Versuche gefahren.

Zum Vergleich wurde ein konventioneller kontinuierlich durchrührter Reaktor (CSTR) mit folgenden Charakteristika verwendet:
Arbeitsvolumen: 4 Liter
Kopfraum: ca. 1 Liter
Rührerdrehzahl: 100 rpm (Umdrehungen pro Minute)
Dieser Laborreaktor ist mit einem Biogasfermenter mit Zentralrührwerk und einem Höhe zu Durchmesser-Verhältnis (H:D) von 1:1 vergleichbar.

Die maximalen Raumbelastungen der geprüften Substrate lagen in diesem Laborreaktor bei:
Maissilagepresssaft: 7 kg oTS/ m³ d
Speisereste: 3 kg oTS/ m³ d (Schaumbildung bei höheren Belastungen)
Zuckerrübe: 7 kg oTS/ m³ d

Bei allen Experimenten mit dem konventionellen Laborreaktor führten Unregelmä-βigkeiten in der Substratzufuhr (schwankende Mengen, Qualität oder so genannte "Shock loads" - plötzliche Überfrachtung mit großen Substratmengen) zu einer starken Verminderung der Biogasproduktion bis zum völligen Abbruch des Biogasprozesses.

### Der erfindungsgemäße Versuchsreaktor

Für die Ableitung der erfindungsrelevanten Parameter wurde ein Versuchsreaktor im verkleinerten Maßstab verwendet, der in seinem Grundaufbau der Darstellung in Figur 1 entspricht. Dieser im vorliegenden Fall aus Acrylglas gefertigte Versuchsreaktor hat ein Arbeitsvolumen von 4 Litern mit vier Kompartimenten (7 (i) - 7 (iv)) mit jeweils einer abwärts und einer aufwärts durchströmten Kammer (8 (i) - 8 (iv); 9 (i) - 9 (iv)). Die Kompartimente (7 (i) - 7 (iv)) haben jeweils eine Grundfläche von 0,002 m². Der Innenraum des Reaktors ist 0,5 m hoch mit einem Gasraum (5) von zusätzlich 0,2 m. Die Volumina der Kammern (8 (i) - 8 (iv); 9 (i) - 9 (iv)) sind abhängig vom jeweiligen Verhältnis von abwärts durchströmter Kammer (8 (i) - 8 (iv)) zu aufwärts durchströmter Kammer (8 (i) - 8 (iv)) und erfindungsgemäß dem jeweiligen Trockensubstanzgehalt des Substrats angepasst. Die entsprechenden Strömungsgeschwindigkeiten in den einzelnen Kammern sind aufgeführt. In einem Bioreaktor im Produktionsmaßstab von mehreren Kubikmetern werden die Strömungsgeschwindigkeiten höher liegen (bis 0,5 m/h).

### Analysenmethoden

Der Trockensubstanzgehalt der Substrate (TS) wurde gravimetrisch durch Trocknung einer Probe bei 105°C über 24 h (bis zum konstanten Gewicht) bestimmt und in Prozent der festen Bestandteile angegeben. Die organische Trockensubstanz (oTS) ist der Glühverlust der getrockneten Probe, der bei der Verglühung der Probe bei 600°C entsteht. Die oTS stellt den prozentualen Anteil an organischer Substanz bezogen auf die Trockensubstanz der Probe dar.

Für die Bestimmung der CSB-Werte wurden Küvettentest-Kits (LCK514) der Fa. Hach-Lange verwendet.

Der Biogasertrag wurde mit einem Gaszähler "Milligascounter" der Fa. Ritter bestimmt.

### Aufbereitung der Substrate

Aufgrund der geringen Durchströmgeschwindigkeiten und der damit verbundenen niedrigen Pumpraten der verwendeten Schlauchpumpen war es notwendig, die Substrate besonders vorzubehandeln. Dazu wurden die Substrate in einer Laborhammermühle mit einem Siebdurchmesser von 0,5mm vermahlen und homogenisiert.

### Ergebnisse der Testfermentationen

Einzelne Testsubstrate wurden im Versuchreaktor bei 37°C und unter Zusatz einer Impfkultur, die aus entgastem Klärschlamm einer Anaerobstufe einer kommunalen Kläranlage stammte, vergoren.
Substrate mit einem Trockensubstanzgehalt von 2 - 5 % (Behandlung mittels erfindungsgemäßem Reaktor und Anwendung des erfindungsgemäßen Verfahrens)
Testsubstrat: Schweinegülle mit 4 % Trockensubstanzgehalt
Verweilzeit: 12 Tage
Verhältnis der abwärts- zur aufwärtsdurchströmten Kammer: 1:2
Strömungsgeschwindigkeit in der abwärtsdurchströmten Kammer: 0,02 m/h
Strömungsgeschwindigkeit in der aufwärtsdurchströmten Kammer: 0,01 m/h
CSB am Reaktoreinlauf: 40.000 - 50.000 mg/l
CSB am Reaktorauslauf: 2.000 - 3.000 mg/l
Raumbelastung: max. 3 kg oTS/m³ d
Biogasertrag: 20m³ je t Schweinegülle

Substrate mit einem Trockensubstanzgehalt von 5 - 10 % (Behandlung mittels erfindungsgemäßem Reaktor und Anwendung des erfindungsgemäßen Verfahrens)
Testsubstrat: thermisch vorbehandelter Maissilagepresssaft mit 9 % Trockensubstanzgehalt
Verweilzeit: 8 Tage
Verhältnis der abwärts- zur aufwärtsdurchströmten Kammer: 1:1
Strömungsgeschwindigkeit in der abwärtsdurchströmten Kammer: 0,02 m/h
Strömungsgeschwindigkeit in der aufwärtsdurchströmten Kammer: 0,02 m/h
CSB am Reaktoreinlauf: 120.000 - 140.000 mg/l
CSB am Reaktorauslauf: 500 - 1.000 mg/l
Raumbelastung: max. 11 kg oTS/m³ d
Biogasertrag: 65m³ je t Maissilagepresssaft
Methangehalt: im gesamten Gasvolumen >70%, im Gasraum der letzten Kammer >90%

Substrate mit einem Trockensubstanzgehalt von 10 - 15 % (Behandlung mittels erfindungsgemäßem Reaktor und Anwendung des erfindungsgemäßen Verfahrens)
Testsubstrat: hygienisierte Speisereste mit 14,5 % Trockensubstanzgehalt
Verweilzeit: 10 Tage
Verhältnis der abwärts- zur aufwärtsdurchströmten Kammer: 2:1
Strömungsgeschwindigkeit in der abwärtsdurchströmten Kammer: 0,012 m/h
Strömungsgeschwindigkeit in der aufwärtsdurchströmten Kammer: 0,025 m/h
CSB am Reaktoreinlauf: 200.000 - 230.000 mg/l
CSB am Reaktorauslauf: 1.000 - 2.000 mg/l
Raumbelastung: max. 15 kg oTS/m³ d
Biogasertrag: 131 m³ je t Speisereste
Methangehalt: im gesamten Gasvolumen >76%, im Gasraum der letzten Kammer >95%

Substrate mit einem Trockensubstanzgehalt von 15 - 20 % (Behandlung mittels erfindungsgemäßem Reaktor und Anwendung des erfindungsgemäßen Verfahrens)
Testsubstrat: zerkleinerte Zuckerrüben mit 19 % Trockensubstanzgehalt
Verweilzeit: 10 Tage
Verhältnis der abwärts- zur aufwärtsdurchströmten Kammer: 3:1
Strömungsgeschwindigkeit in der abwärtsdurchströmten Kammer: 0,01 m/h
Strömungsgeschwindigkeit in der aufwärtsdurchströmten Kammer: 0,033 m/h
CSB am Reaktoreinlauf: 270.000 - 300.000 mg/l
CSB am Reaktorauslauf: 1.000 - 2.000 mg/l
Raumbelastung: max. 20 kg oTS/m³ d
Biogasertrag: 153m³ je t Zuckerrüben
Methangehalt: im gesamten Gasvolumen >60%, im Gasraum der letzten Kammer >85%

Beim erfindungsgemäßen Verfahren zeigt sich in allen Fällen eine Unempfindlichkeit gegenüber Substratschwankungen, sowohl in Bezug auf die Menge wie auch in Bezug auf die Qualität des Substrates.

Der erfindungsgemäße Reaktor ist durch die Kompartimentierung robust gegen Schwankungen und Störungen. Dadurch sind im erfindungsgemäßen Reaktor höhere Raumbelastungen möglich. In den einzelnen Kompartimenten stellen sich unterschiedliche Fermentationsbedingungen (wie z.B. pH-Werte) ein, die zur Stabilisierung des Fermentationsprozesses führen. Der Biogasprozess besteht aus verschiedenen Einzelschritten, die zwar aufeinander aufbauen aber unter unterschiedlichen Bedingungen optimal ablaufen. Der erfindungsgemäße Reaktor unterstützt speziell diese Prozessbesonderheiten. Die höheren Raumbelastungen, kürzeren Verweilzeiten und die besseren Fermentationsbedingungen führen zu insgesamt höheren Erträgen pro Zeiteinheit (erhöhte Raum-Zeit-Ausbeute) verglichen mit konventionellen Reaktoren, wie der nachfolgenden Tabelle 2 zu entnehmen ist.

**Tabelle 2: Erhöhung der Raum-Zeit-Ausbeute des erfindungsgemäßen Laborreaktors im Vergleich zum konventionellen Laborreaktor:**

| Testsubstrat | Raum-Zeit-Ausbeute [m³ Biogas je m³ Reaktorvolumen und Tag] | | Erhöhung der Raum-Zeit-Ausbeute |
|---|---|---|---|
| | erfindungsgemäßer Laborreaktor | konventioneller Laborreaktor | |
| Schweinegülle | 2 | 0,7 | 3 x |
| vorbehandelter Maissilagepresssaft | 9 | 3 | 3 x |
| hygienisierte Speisereste | 14 | 2 | 7 x |
| zerkleinerte Zuckerrüben | 16 | 4 | 4 x |

### Bezugszeichenliste:

- 1: Zulauf
- 2 (i) bis 2 (iv): Gasausgänge
- 3: Ablauf
- 4: Boden
- 5 (i) bis 5 (iv): Gasräume
- 6: Trennwände
- 7 (i) bis 7 (iv): Kompartimente (i) bis (iv)
- 8 (i) bis 8 (iv): abwärts durchströmte Kammern in den Kompartimenten (i) bis (iv)
- 9 (i) bis 9 (iv): aufwärts durchströmte Kammern in den Kompartimenten (i) bis (iv)
- 10 (i) bis 10 (iv): Gaseintritte

## Patentansprüche

1. Verfahren zur Fermentation eines fließfähigen Substrats mit definiertem Trockensubstanzgehalt, unter Verwendung eines Reaktors, wobei das Verfahren mindestens die Verfahrensschritte aufweist:
□ Zuführen des Substrates durch mindestens einen Zulauf (1) des Reaktors,
□ Hindurchführen des Substrates durch den Reaktor, wobei der Reaktor eine Mehrzahl von Trennwänden (6) aufweist, die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,
□ Abführen des fermentierten Substrates aus mindestens einen Ablauf (3) des Reaktors,
**dadurch gekennzeichnet, dass**
▪ zur Erhöhung oder Reduktion des Verhältnisses
- des Volumens der in die eine Richtung vom Substrat durchströmten Kammern (8 (i) - 8 (iv))
- zu dem Volumen der in die andere Richtung vom Substrat durchströmten Kammern (9 (i) - 9 (iv))
zumindest ein Teil der Trennwände (6) in ihrer räumlichen Lage und/oder Position und/oder Ausdehnung bewegt werden,
wobei die Bewegung und/oder Ausdehnung der Trennwände (6) in Abhängigkeit vom Trockensubstanzgehalt des fließfähigen Substrats gesteuert wird,
▪ jedes Kompartiment (7 (i) - 7 (iv)) ein für das Substrat bestimmtes Innenvolumen und einen zugeordneten Gasraum (5 (i) - 5 (iv)) aufweist,
▪ mindestens ein Gasraum von mindestens einem Kompartiment, ausgesucht aus allen Gasräumen (5 (i) - 5 (iv)) aller Kompartimente (7 (i) - 7 (iv)), getrennt ausgebildet ist von dem restlichen Gasraum oder den restlichen Gasräumen (5 (i) - 5 (iv)) der restlichen Kompartimente (7 (i) - 7 (iv)),
und wobei das Verfahren mindestens die folgenden weiteren Verfahrensschritte aufweist:
▪ Abführen von Prozessgas aus dem mindestens einen Gasraum, getrennt ausgebildet von dem restlichen Gasraum oder den restlichen Gasräumen (5 (i) - 5 (iv)) der restlichen Kompartimente (7 (i) - 7 (iv)),
▪ Zuführen von zuvor abgeführtem Prozessgas und/oder Fremdgas in das für das Substrat bestimmte Innenvolumen von mindestens einem der ausgebildeten Kompartimente (7 (i) - 7 (iv)).

2. Verfahren nach Patentanspruch 1, **dadurch gekennzeichnet, dass** Prozessgas jeweils des nachfolgenden Kompartiments (7 (i) - 7 (iv)) in das Innenvolumen des vorherigen Kompartiments (7 (i) - 7 (iv)) eingeführt wird.

3. Verfahren nach einem der Patentansprüche 1 oder 2, **dadurch gekennzeichnet, dass** ein Sauerstoff enthaltendes Gas als Fremdgas in das Innenvolumen des ersten vom Substrat passierten Kompartiments (7 (i)) eingeführt wird.

4. Verfahren nach einem der Patentansprüche 1 bis 3, **dadurch gekennzeichnet, dass** die Zuführung von Prozessgas und/oder Fremdgas mittels eines Gasstromes erfolgt, dessen Stromrichtung in Gegenstromrichtung des Substrates ausgerichtet ist.

5. Verfahren nach einem der Patentansprüche 1 bis 4, **dadurch gekennzeichnet, dass** der Reaktor entlang seiner Längsachse eine Mehrzahl nebeneinander positionierter Kompartimente (7 (i) - 7 (iv)) aufweist.

6. Verfahren nach Patentanspruch 5, **dadurch gekennzeichnet, dass** jedes einzelne Kompartiment (7 (i) - 7 (iv)) jeweils in zwei gegenläufig vom Substrat durchströmte Kammern (8 (i) - 8 (iv); 9 (i) - 9 (iv)) unterteilt ist, von denen die Kammern (8 (i) - 8 (iv)) vom Substrat abwärts und die Kammern (9 (i) - 9 (iv)) vom Substrat aufwärts durchströmt werden.

7. Verfahren nach einem der Patentansprüche 5 bis 6, **dadurch gekennzeichnet, dass** das Verhältnis des jeweiligen Volumens der abwärts vom Substrat durchströmten Kammern (8 (i) - 8 (iv) zu dem Volumen der aufwärts vom Substrat durchströmten Kammern (9 (i) - 9 (iv) mit steigendem Trockensubstanzgehalt des Substrates zunimmt.

8. Reaktor zur Fermentation eines fließfähigen Substrats mit definiertem Trockensubstanzgehalt, mindestens aufweisend:
□ einen Zulauf (1),
□ einen Ablauf (3),
□ eine Mehrzahl von Trennwänden (6), die mindestens das für das Substrat bestimmte Innenvolumen des Reaktors in eine Mehrzahl von Kompartimenten (7 (i) - 7 (iv)) und jedes einzelne Kompartiment (7 (i) - 7 (iv)) jeweils in mindestens zwei gegenläufig vom Substrat durchströmte Kammern (8 (i) - 8 (iv); 9 (i) - 9 (iv)) unterteilen,
**dadurch gekennzeichnet, dass**
▪ zur Erhöhung oder Reduktion des Verhältnisses
- des Volumens der in die eine Richtung vom Substrat durchströmten Kammern (8 (i) - 8 (iv)
- zu dem Volumen der in die andere Richtung vom Substrat durchströmten Kammern (9 (i) - 9 (iv)
zumindest ein Teil der Trennwände (6) in ihrer räumlichen Lage und/oder Position beweglich angeordnet sind,
und dass der Reaktor mindestens eine Steuerung zur Bewegung der Trennwände (6) in Abhängigkeit vom Trockensubstanzgehalt des fließfähigen Substrats aufweist,
▪ jedes Kompartiment (7 (i) - 7 (iv)) ein für das Substrat bestimmte Innenvolumen und einen zugeordneten Gasraum (5 (i) - 5 (iv)) aufweist,
▪ mindestens ein Gasraum von mindestens einem Kompartiment, ausgesucht aus allen Gasräumen (5 (i) - 5 (iv)) aller Kompartimente (7 (i) - 7 (iv)), getrennt ausgebildet ist von dem restlichen Gasraum oder den restlichen Gasräumen (5 (i) - 5 (iv)) der restlichen Kompartimente (7 (i) - 7 (iv)),
▪ der mindestens eine Gasraum einen Gasausgang zur Abführung von Prozessgas aufweist,
▪ mindestens ein Kompartiment (7 (i) - 7 (iv)) einen Gaseintritt (10 (i) - 10 (iv)) zur Zuführung von Prozessgas und/oder Fremdgas aufweist.

9. Reaktor nach Patentanspruch 8, **dadurch gekennzeichnet, dass** jedes Kompartiment (7 (i) - 7 (iv)) ein für das Substrat bestimmtes Innenvolumen und einen eigenen Gasraum (5 (i) - 5 (iv)) aufweist, wobei dieser eigene Gasraum von den Gasräumen der anderen Kompartimente (7 (i) - 7 (iv)) getrennt ausgebildet ist.

10. Reaktor nach Patentanspruch 9, **dadurch gekennzeichnet, dass** jeder Gasraum (5 (i) - 5 (iv)) von jedem Kompartiment (7 (i) - 7 (iv)) einen ausschließlich ihm zugeordneten Gasausgang (2 (i) - 2 (iv)) zur Abführen von Prozessgas aus diesem Gasraum (5 (i) - 5 (iv)) aufweist.

11. Reaktor nach einem der Patentansprüche 8 bis 10, **dadurch gekennzeichnet, dass** der Reaktor entlang seiner Längsachse eine Mehrzahl nebeneinander positionierter Kompartimente (7 (i) - 7 (iv)) aufweist.

12. Reaktor nach Patentanspruch 11, **dadurch gekennzeichnet, dass** jedes einzelne Kompartiment (7 (i) - 7 (iv)) jeweils in zwei gegenläufig vom Substrat durchströmte Kammern (8 (i) - 8 (iv); 9 (i) - 9 (iv)) unterteilt ist, von denen die Kammern (8 (i) - 8 (iv)) vom Substrat abwärts und die Kammern (9 (i) - 9 (iv)) vom Substrat aufwärts durchströmt sind.

13. Reaktor nach Patentanspruch 12, **dadurch gekennzeichnet, dass** der Gaseintritt (10 (i) - 10 (iv)) zur Zuführung von Prozessgas und/oder Fremdgas am Boden der vom Substrat abwärts durchströmten Kammern (8 (i) - 8 (iv)) ausgebildet ist.

14. Reaktor nach einem der Patentansprüche 11 bis 13, **dadurch gekennzeichnet, dass** zumindest ein Teil der Trennwände (6) in Richtung der Längsachse des Reaktors verschiebbar angeordnet sind.

15. Reaktor nach einem der Patentansprüche 11 bis 14, **dadurch gekennzeichnet, dass** sich die Trennwände (6) über die gesamte Breite des Reaktors erstrecken.

## Claims

1. Process for fermenting a free-flowing substrate with a given dry substance content, using a reactor, the process having at least the following process steps:
- supplying the substrate via at least one feed inlet (1) of the reactor,
- conveying the substrate through the reactor, the reactor having a plurality of partitions (6) which divide at least the internal volume of the reactor intended for the substrate into a plurality of compartments (7(i)-7(iv)) and each single compartment (7(i)-7(iv)) in each case into at least two chambers (8(i)-8(iv); 9(i)-9(iv)) passed through by the substrate in opposite directions,
- drawing off the fermented substrate via at least one discharge outlet (3) of the reactor,
**characterized in that**
• in order to increase or reduce the ratio
- of the volume of the chambers (8(i)-8(iv)) passed through by the substrate in one direction
- to the volume of the chambers (9(i)-9(iv)) passed through by the substrate in the other direction
at least some of the partitions (6) are moved with regard to their spatial location and/or position and/or extension,
the movement and/or extension of the partitions (6) being controlled depending on the dry substance content of the free-flowing substrate,
• each compartment (7(i)-7(iv) has an internal volume intended for the substrate and an assigned gas space (5(i)-5(iv)),
• at least one gas space of at least one compartment, selected from all the gas spaces (5(i)-5(iv)) of all the compartments (7(i)-7(iv)), is formed separately from the remaining gas space or the remaining gas spaces (5(i)-5(iv)) of the remaining compartments (7(i)-7(iv)),
and the process having at least the following further process steps:
• drawing off process gas from the at least one gas space, formed separately from the remaining gas space or the remaining gas spaces (5(i)-5(iv)) of the remaining compartments (7(i)-7(iv)),
• supplying the previously drawn-off process gas and/or foreign gas into the internal volume of at least one of the formed compartments (7(i)-7(iv)), which is intended for the substrate.

2. Process according to Claim 1, **characterized in that** the process gas of each following compartment (7(i)-7(iv)) is introduced into the internal volume of the preceding compartment (7(i)-7(iv).

3. Process according to either one of Claims 1 or 2, **characterized in that** an oxygen-containing gas is introduced as a foreign gas into the internal volume of the first compartment (7(i)) passed through by the substrate.

4. Process according to one of Claims 1 to 3, **characterized in that** supplying of the process gas and/or foreign gas is performed by means of a gas stream, which has a direction of flow in the opposite direction to that of the substrate.

5. Process according to one of Claims 1 to 4, **characterized in that** the reactor has a plurality of compartments (7(i)-7(iv) positioned next to each other along its longitudinal axis.

6. Process according to Claim 5, **characterized in that** each single compartment (7(i)-7(iv)) is divided in each case into two chambers (8(i)-8(iv); 9(i)-9(iv)) passed through by the substrate in opposite directions, the chambers (8(i)-8(iv)) thereof being passed through by the substrate in a downwards direction and the chambers (9(i)-9(iv)) thereof being passed through by the substrate in an upwards direction.

7. Process according to either one of Claims 5 or 6, **characterized in that** the ratio of the respective volume of the chambers (8(i)-8(iv)) passed through by the substrate in a downwards direction to the volume of the chambers (9(i)-9(iv)) passed through by the substrate in an upwards direction increases with an increase in the dry substance content of the substrate.

8. Reactor for fermenting a free-flowing substrate with a given dry substance content, having at least:
- a feed inlet (1),
- a discharge outlet (3)
- a plurality of partitions (6) which divide the internal volume of the reactor intended for the substrate into a plurality of compartments (7(i)-7(iv)) and each single compartment (7(i)-7(iv)) in each case into at least two chambers (8(i)-8(iv); 9(i)-9(iv)) passed through by the substrate in opposite directions,
**characterized in that**
• in order to increase or reduce the ratio
- of the volume of the chambers (8(i)-8(iv)) passed through by the substrate in one direction
- to the volume of the chambers (9(i)-9(iv)) passed through by the substrate in the other direction,
at least some of the partitions (6) are arranged movably with regard to their spatial location and/or position, and that the reactor has at least one control system for moving the partitions (6) depending on the dry substance content of the free-flowing substrate,
• each compartment (7(i)-7(iv) has an internal volume intended for the substrate and an assigned gas space (5(i)-5(iv)),
• at least one gas space of at least one compartment, selected from all the gas spaces (5(i)-5(iv)) of all the compartments (7(i)-7(iv)), is formed separately from the remaining gas space or the remaining gas spaces (5(i)-5(iv)) of the remaining compartments (7(i)-7(iv)),
• the at least one gas space has a gas outlet for drawing off process gas,
• the at least one compartment (7(i)-7(iv)) has a gas inlet (10(i)-10(iv) for supplying the process gas and/or foreign gas.

9. Reactor according to Claim 8, **characterized in that** each compartment (7(i)-7((iv)) has an internal volume intended for the substrate and an associated gas space (5(i)-5(iv)), this associated gas space being formed separately from the gas spaces of the other compartments (7(i)-7(iv)).

10. Reactor according to Claim 9, **characterized in that** each gas space (5(i)-5(iv)) of each compartment (7(i)-7(iv)) has a gas outlet (2(i)-2(iv)) assigned exclusively to it for drawing off process gas from the gas space (5(i)-5(iv)).

11. Reactor according to one of Claims 8 to 10, **characterized in that** the reactor has a plurality of compartments (7(i)-7(iv) positioned next to each other along its longitudinal axis.

12. Reactor according to Claim 11, **characterized in that** each single compartment (7(i)-7(iv)) is divided in each case into two chambers (8(i)-8(iv); 9(i)-9(iv)) passed through by the substrate in opposite directions, the chambers (8(i)-8(iv)) thereof being passed through by the substrate in a downwards direction and the chambers (9(i)-9(iv)) thereof being passed through by the substrate in an upwards direction.

13. Reactor according to Claim 12, **characterized in that** the gas inlet (10(i)-10(iv)) for supplying process gas and/or foreign gas is formed on the bottom of the chambers (8(i)-8(iv)) passed through by the substrate in a downwards direction.

14. Reactor according to one of Claims 11 to 13, **characterized in that** at least some of the partitions (6) are arranged displaceably in the direction of the longitudinal axis of the reactor.

15. Reactor according to one of Claims 11 to 14, **characterized in that** the partitions (6) extend over the entire width of the reactor.

## Revendications

1. Procédé pour la fermentation d'un substrat apte à s'écouler présentant une teneur en substances sèches définie en utilisant un réacteur, le procédé ayant au moins les étapes suivantes :
□ introduction du substrat par au moins une alimentation (1) du réacteur,
□ passage du substrat à travers le réacteur, le réacteur étant muni d'une pluralité de parois de séparation (6) qui scindent au moins le volume intérieur du réacteur destiné au substrat en une pluralité de compartiments (7 (i) - 7 (iv)) et chacun des compartiments (7 (i) - 7 (iv)) en au moins deux chambres (8 (i) - 8 (iv) ; 9 (i) - 9 (iv)) traversées en sens opposé par le substrat,
□ évacuation du substrat fermenté par au moins une évacuation (3) du réacteur,
**caractérisé en ce que**
▪ pour augmenter ou réduire le rapport entre
- le volume des chambres (8 (i) - 8 (iv)) traversées dans un sens par le substrat et
- le volume des chambres (9 (i) - 9 (iv)) traversées par le substrat dans l'autre sens
au moins une partie des parois de séparation (6) sont déplacées du point de vue de leur situation spatiale et/ou leur position et/ou leurs dimensions,
le déplacement et/ou la dimension des parois de séparation (6) étant commandé en fonction de la teneur en substances sèches dans le substrat apte à s'écouler,
▪ chaque compartiment (7 (i) - 7 (iv)) présente un volume interne destiné au substrat et une chambre de gaz (5 (i) - 5 (iv) associée,
▪ au moins une chambre de gaz d'au moins un compartiment, choisie parmi toutes les chambres (5 (i) - 5 (iv)) de tous les compartiments (7 (i) - 7 (iv)), est conçue séparée de l'autre chambre de gaz ou des autres chambres de gaz (5 (i) - 5 (iv)) des autres compartiments (7 (i) - 7 (iv)),
et le procédé comporte au moins les étapes supplémentaires suivantes
▪ extraction du gaz de procédé hors de la au moins une chambre de gaz conçue séparée de l'autre chambre de gaz ou des autres chambre de gaz (5 (i) - 5 (iv)) des autres compartiments (7 (i) - 7 (iv)),
▪ introduction du gaz de procédé extrait préalablement et/ou d'un gaz étranger dans le volume intérieur destiné au substrat d'au moins un des compartiments formés (7 (i) - 7 (iv)).

2. Procédé selon la revendication 1, **caractérisé en ce que** le gaz de procédé du compartiment suivant (7 (i) - 7 (iv)) est introduit dans le volume intérieur du compartiment (7 (i) - 7 (iv)) précédent.

3. Procédé selon l'une des revendications 1 ou 2, **caractérisé en ce qu'**un gaz contenant de l'oxygène est introduit en tant que gaz étranger dans le volume intérieur du compartiment (7 (i)) traversé en premier par le substrat.

4. Procédé selon l'une des revendications 1 à 3, **caractérisé en ce que** l'introduction du gaz de procédé et/ou du gaz étranger est réalisée au moyen d'un courant gazeux dont la direction du courant est dirigée dans la direction inverse de celle du substrat.

5. Procédé selon l'une des revendications 1 à 4, **caractérisé en ce que** le réacteur présente le long de son axe longitudinal une pluralité de compartiments (7 (i) - 7 (iv)) positionnés les uns à côté des autres.

6. Procédé selon la revendication 5, **caractérisé en ce que** chacun des compartiments (7 (i) - 7 (iv)) est scindé en deux chambres (8 (i) - 8 (iv) ; 9 (i) - 9 (iv)) traversées par le substrat dans des sens opposés, les chambres (8 (i) - 8 (iv)) étant traversées vers le bas par le substrat et les chambres (9 (i) - 9 (iv)) étant traversées vers le haut par le substrat.

7. Procédé selon l'une des revendications 5 à 6, **caractérisé en ce que** le rapport entre chacun des volumes des chambres (8 (i) - 8 (iv)) traversées vers le bas par le substrat et le volume des chambres (9 (i) - 9 (iv)) traversées vers le haut par le substrat augmente avec l'augmentation de la teneur en substances sèches dans le substrat.

8. Réacteur pour la fermentation d'un substrat apte à s'écouler présentant une teneur en substances sèches définie, lequel réacteur présente au moins :
□ une alimentation (1),
□ une évacuation (3),
□ une pluralité de parois de séparation (6) qui scindent au moins le volume intérieur du réacteur destiné au substrat en une pluralité de compartiments (7 (i) - 7 (iv)) et chacun des compartiments (7 (i) - 7 (iv)) en au moins deux chambres (8 (i) - 8 (iv) ; 9 (i) - 9 (iv)) traversées en sens opposé par le substrat,
**caractérisé en ce que**
▪ pour augmenter ou réduire le rapport entre
- le volume des chambres (8 (i) - 8 (iv)) traversées dans un sens par le substrat et
- le volume des chambres (9 (i) - 9 (iv)) traversées par le substrat dans l'autre sens
au moins une partie des parois de séparation (6) sont disposées mobiles du point de vue de leur situation spatiale et/ou leur position,
et **en ce que** le réacteur comprend au moins une commande pour déplacer les parois de séparation (6) en fonction de la teneur en substances sèches dans le substrat apte à s'écouler,
▪ chaque compartiment (7 (i) - 7 (iv)) présente un volume interne destiné au substrat et une chambre de gaz (5 (i) - 5 (iv) associée,
▪ au moins une chambre de gaz d'au moins un compartiment, choisie parmi toutes les chambres (5 (i) - 5 (iv)) de tous les compartiments (7 (i) - 7 (iv)), est conçue séparée de l'autre chambre de gaz ou des autres chambres de gaz (5 (i) - 5 (iv)) des autres compartiments (7 (i) - 7 (iv)),
▪ la au moins une chambre de gaz présentant une sortie de gaz pour évacuer le gaz de procédé,
▪ au moins un compartiment (7 (i) - 7 (iv)) présentant une entrée de gaz (10 (i) - 10 (iv)) pour l'introduction du gaz de procédé et/ou d'un gaz étranger.

9. Réacteur selon la revendication 8, **caractérisé en ce que** chacun des compartiments (7 (i) - 7 (iv)) présente un volume interne destiné au substrat et sa propre chambre de gaz (5 (i) - 5 (iv)), ces propres chambres de gaz étant conçues séparées des chambres de gaz des autres compartiments (7 (i) - 7 (iv)).

10. Réacteur selon la revendication 9, **caractérisé en ce que** chaque chambre de gaz (5 (i) - 5 (iv)) de chacun des compartiments (7 (i) - 7 (iv)) présente une sortie de gaz (2 (i) - 2 (iv)) qui lui est exclusivement attribuée pour extraire le gaz de procédé de la chambre de gaz (5 (i) - 5 (iv)).

11. Réacteur selon l'une des revendications 8 à 10, **caractérisé en ce que** le réacteur présente le long de son axe longitudinal une pluralité de compartiments (7 (i) - 7 (iv)) positionnés les uns à côté des autres.

12. Réacteur selon la revendication 11, **caractérisé en ce que** chacun des compartiments (7 (i) - 7 (iv)) est scindé en deux chambres (8 (i) - 8 (iv) ; 9 (i) - 9 (iv)) traversées par le substrat dans des sens opposés, les chambres (8 (i) - 8 (iv)) étant traversées vers le bas par le substrat et les chambres (9 (i) - 9 (iv)) étant traversées vers le haut par le substrat.

13. Réacteur selon la revendication 12, **caractérisé en ce que** l'entrée de gaz (10 (i) - 10 (iv)) pour introduire le gaz de procédé et/ou le gaz étranger est formée dans le fond des chambres (8 (i) - 8 (iv)) traversées vers le bas par le substrat.

14. Réacteur selon l'une des revendications 11 à 13, **caractérisé en ce qu'**au moins une partie des parois de séparation (6) sont disposées mobiles en direction de l'axe longitudinal du réacteur.

15. Réacteur selon l'une des revendications 11 à 14, **caractérisé en ce que** les parois de séparation (6) s'étendent sur toute la largeur du réacteur.
